# EUROPÄISCHE PATENTANMELDUNG

(11) **EP 1 716 872 A1**
(43) Veröffentlichungstag der Anmeldung: **02.11.2006**
(21) Anmeldenummer: 05009336.8
(22) Anmeldetag: 28.04.2005
(51) Int. Cl.: A61L 9/12, A61L 9/04

(54) **Temperatur reguliertes Stoffdispensersystem**

(71) Anmelder: Mibelle AG Cosmetics, 5033 Buchs (CH)
(72) Erfinder: Marte, Walter, 9631 Ulisbach (SG) (CH); Lüthi, Walter, 9642 Ebnat-Kappel (SG) (CH); Hübner, Hans-Jürgen, 6645 Brione S.M. (TI) (CH); Zimmermann, Michael, 5033 Buchs (CH)
(74) Vertreter: Forstmeyer, Dietmar

(57) **Zusammenfassung**

Die Erfindung betrifft ein chemisch/mechanisches, sich eigendynamisch regulierendes Dispensersystem für Stoffe, insbesondere für Duftstoffe und/oder Arzneimittel, welches die Wirkstoffe aufgrund des enthaltenen Polymers mit hoher Schmelzenthalpie und ggf. einer niedermolekularen wasserlöslichen Substanz mit endothermer Lösungs- bzw. Sorptionsenthalpie trotz erhöhter Temperatur gleichmäßig abgibt. Zusätzlich gibt die Erfindung die Möglichkeit, die Abgabe von Wirkstoffen bei Überschreiten einer bestimmten Temperatur zu unterbinden.

## Beschreibung

### Einleitung und Gebiet der Erfindung

Die Erfindung betrifft ein chemisch/mechanisches, sich eigendynamisch regulierendes Dispensersystem für Stoffe, insbesondere für Duftstoffe und/oder Arzneimittel, dessen Anwendung gleichermassen in Wohn- und Arbeitsräumen aber auch in Autos und Klimaanlagen aller Art sowie in öffentlichen Gebäuden und im Hotel- und Krankenhausbereich möglich ist. Die Erfindung umfasst ferner einen Stoffblister mit einem Wirkstoff sowie einen Druckblister zum Verschliessen des Stoffdispensersystems.

Die Zielsetzung, die mit dem Einsatz von Stoffdispensern, insbesondere von Duftdispensern, verbunden ist, ist die Verbreitung von allgemeinem Wohlbefinden. Dies schliesst sowohl die Abgabe von "Wohlgerüchen" ein als auch die Abgabe von Substanzen zur Eliminierung unerwünschter Gerüche. Während Geschmacksstoffe bezüglich ihres Molekulargewichtes und ihrer Polarität keine Begrenzung kennen, wird die Wahrnehmung von Duftstoffen meist durch relativ kleine, hydrophobe und stark flüchtige Substanzen ausgelöst. Sehr wesentlich ist die Feststellung, dass die Reizauslösung durch Duftstoffe (= Riechstoffe) bereits bei extrem niedrigen Schwellenwerten eintritt, im Gegensatz zu den Geschmacksstoffen (G. Ohloff, Düfte, Verlag Helvetica Chimica Acta AG Zürich, 2004). Durch erhöhte Duftstoffkonzentrationen in der Luft werden die Rezeptormembranen, die als biochemische Detektoren fungieren, für längere Zeit blockiert und anstelle von Wohlbefinden kann Übelkeit und Ekel ausgelöst werden.

### Stand der Technik

Die heute in den verschiedensten Bereichen eingesetzten Duftstoffdispensersysteme zeigen in ihrem Design eine grosse Vielfalt. Häufig anzutreffende Systeme sind Blisterpackungen, die den Duftstoff als Lösung oder mit nicht flüchtigen Chemikalien in verdicktem Zustand, d.h. als Gel, enthalten (z.B. Cinnamon Dream von Migros, Schweiz). Andere Systeme, die häufig in Autos anzutreffen sind, enthalten den Duftstoff als Lösung in einem 5 - 50 ml Speicherbehälter, wobei der Duftstoff z.B. über ein poröses Dochtsystem an die Umgebungsluft abgegeben wird. Zur Stoffabgaberegulierung enthalten diese Systeme vielfach einen mechanischen, von Hand zu betätigenden Verschluss. Weitere, die Stoffabgabe fördernde Systeme enthalten Ventilatoren, bzw. werden in Ventilationskanälen eingebaut. Wiederum andere Duftsysteme bestehen aus einem festen mit Duftstoff imprägnierten Träger, der diesen, ähnlich den eben geschilderten, weitgehend unkontrolliert in die angrenzende Atmosphäre abgibt.

Allen diesen Systemen gemeinsam ist die mehr oder weniger unkontrollierte Stoffabgabe in Abhängigkeit von der Temperatur, d.h. es wird um so mehr Duftstoff abgegeben, je höher die Umgebungstemperatur ist. Dies hat zur Folge, dass in Abhängigkeit der das Duftsystem umgebenden Lufttemperatur hohe Duftstoffabgaberaten erreicht werden, in deren Folge anstelle der "Wellness" verbreitenden Funktion Übelkeit und allergische Reaktionen resultieren. Ein weiterer Nachteil bestehender Systeme ist die temperaturbedingte, viel zu rasche Entleerung des Duftstoffspeichers bzw. Duftstoffbehälters (auch Duftstoffblister oder Stoffblister genannt) mit den entsprechenden finanziellen Konsequenzen. Äusserst problematisch wirken sich die erwähnten Nachteile bei sommerlichen Temperaturen aus, da beispielsweise je nach Sonneneinstrahlung in geschlossenen Räumen (z.B. Autos) bis zu 70 °C erreicht werden. Um solchen Extremsituationen zu begegnen, werden typischerweise in Autos mechanisch verschliessbare Duftstoffdispensersysteme eingesetzt. Der wesentliche Nachteil dieser Systeme ist der von Hand zu betätigende und nicht automatisch durch die Umgebungstemperatur kontrollierte Verschlussmechanismus.

Die wichtigsten Hersteller von Wirkstoff-Dispensersystemen sind: Reckitt-Benckiser (Marke: Airwick), Sara Lee (Marke: Ambi Pur), SC Johnson (Marke Glade), Duco Magic Tree, Carfreshener, und Holts.

### Aufgabe und Lösung der Erfindung

Aufgabe der Erfindung ist es, ein automatisch funktionierendes Duftstoffdispensersystem bereitzustellen, das ― relativ unabhängig von der jeweiligen Umgebungstemperatur ― automatisch stets eine bestimmte, konstante Menge an Duftstoff gleichmässig abgibt. Als Option soll darüber hinaus bei Überschreiten einer bestimmten Umgebungstemperatur automatisch die Abgabe von Duftstoffen unterbunden werden, ohne dass es dazu eines menschlichen Eingriffes bedarf.

Die Aufgabe wird durch Anwendung unterschiedlicher physikalisch-chemischer Prinzipien gelöst. Zunächst wird die gleichbleibende Abgabe von Duftstoffen trotz steigender Umgebungstemperatur durch einen im Duftstoffblister vorhandenen "Temperaturpuffer" aus verschiedenen Substanzen bewirkt, der eine zu starke Aufheizung des Systems verhindert. Zusätzlich wird durch einen automatischen, auf einem thermodynamisch - mechanischen Funktionsprinzip basierenden Verschlussmechanismus die Abgabe von Duftstoffen bei Überschreiten einer bestimmten Umgebungstemperatur durch einen Druckblister unterbunden.

Beide Mechanismen der vorliegenden Erfindung, nämlich die trotz erhöhter Aussentemperatur konstante Abgabe von Wirkstoff aufgrund eines "Temperaturpuffers" im Stoffblister und die Verhinderung der Abgabe von Wirkstoff bei Überschreiten einer bestimmten Temperatur mittels eines Druckblisters, können sowohl unabhängig von einander als auch in Kombination eingesetzt werden. Die Kombination beider Vorrichtungen gestattet eine vollkommen automatische Steuerung der Abgabe der Duftstoffe, ohne dass es dazu eines menschlichen Eingriffs bedarf.

Das Problem der Abgabe einer bestimmten konstanten Menge an Duftstoff (= Riechstoff) - in verhältnismässig unabhängiger Weise von der Umgebungstemperatur - wird durch die Verwendung von bei niedriger Temperatur, bevorzugt bei 23 - 40 °C schmelzenden, tendentiell lipophilen Polymeren mit einem HLB-Wert von 8 bis 18 (Hydrophilic-Lipophilic-Balance, siehe H. Sonntag, Lehrbuch der Kolloidwissenschaft, VEB Deutscher Verlag der Wissenschaften Berlin 1977) mit einer hohen Schmelzenthalpie in der Duftstoffmischung gelöst. Durch den Einsatz derartiger Polymere mit hoher Schmelzenthalpie kommt es bei einem Anstieg der Umgebungstemperatur zu einer Verzögerung des Temperaturanstiegs in der Duftstoffmischung, so dass die Polymere als "Temperaturpuffer" im System wirken und dadurch die Abgabe der Duftstoffe trotz steigender Umgebungstemperatur begrenzt wird.

In derselben Richtung wirkt auch die Zugabe von wasserlöslichen organischen, meist polymeren Substanzen und/oder Elektrolyten mit endothermer Sorptions- bzw. Lösungsenthalpie in Wasser. Indem diese Substanzen bei Kontakt mit Wasser bzw. einer Erhöhung des Wasseranteils in der Duftstoffmischung mit einer Temperaturerniedrigung reagieren, fungieren sie ebenfalls als "Temperaturpuffer" und tragen somit ebenfalls zur Lösung des erstgenannten Problems bei, d.h. sie begrenzen die Abgabe von Duftstoffen trotz steigender Umgebungstemperatur. Die Luft kann um so mehr Wasser als Dampf aufnehmen, je höher die Lufttemperatur ist. Bei erhöhter Lufttemperatur steigt also in der Regel der Wassergehalt der Luft an. Die vorstehend genannten Zusätze bewirken, dass bei erhöhten Temperaturen durch die Wassersorption in der Duftstoff-Blistermasse eine Temperatursenkung erfolgt. Dieser Mechanismus funktioniert auch in umgekehrter Richtung. Da die beschriebene Temperaturpufferung die Funktion einer nur beschränkt zur Verfügung stehenden Schmelz- oder Lösungsenthalpie der Duftstoffspeichermasse ist, kann als sinnvolle Massnahme bei langfristig erhöhten oder weiter steigenden Temperaturen eine Verschliessung des Duftstoffblisters erfolgen.

Das Problem einer Verhinderung der Abgabe von Duftstoffen bei Überschreiten einer bestimmten Temperatur wird gelöst, indem eine leichtflüchtige Substanz in ein geschlossenes, ausdehnungsfähiges Gefäss gefüllt wird, welches sich mit steigender Temperatur aufgrund des zunehmenden Dampfdrucks dieser Substanz ausdehnt und dadurch die Öffnung für die Abgabe der Duftstoffe bei Überschreiten einer bestimmten Temperatur verschliesst.

### Ausführungsformen der Erfindung

Die vorliegende Erfindung umfasst jede Kombination der nachfolgenden Merkmale bzw. Ausführungsformen.

Eine Ausführungsform dieser Erfindung betrifft einen Druckblister, der a) ein in mindestens einer Dimension ausdehnungsfähiges Behältnis umfaßt, das b) mindestens ein niedrig siedendes Lösungsmittel enthält.

Gemäß einer weiteren bevorzugten Ausführungsform enthält das Lösungsmittel b) ein Verdickungsmittel und/oder ein Sorptionsmittel.

Gemäß einer weiteren bevorzugten Ausführungsform enthält das Verdickungsmittel ein Cellulosederivat und/oder Polyvinylpyrrolidon, und/oder enthält das Sorptionsmittel chemisch modifizierte Siliciumdioxid-Nanopartikel, wie z.B. methylierte Siliziumdioxid-Nanopartikel "Aerosil R 972" der Firma Degussa.

Gemäß einer weiteren bevorzugten Ausführungsform enthält das Verdickungsmittel eine Hydroxypropyl-Methylcellulose und/oder eine Ethylhydroxyethyl-Cellulose.

Eine Ausführungsform dieser Erfindung betrifft einen Stoffblister mit c) mindestens einer porösen Matrix, und d) mindestens einem Duftstoff und/oder einem Arzneimittel, und e) mindestens einem Polymer, dessen Schmelz- oder Zersetzungstemperatur über 100 °C liegt, und/oder f) mindestens einem Polymer, das bei einer Temperatur von 23 - 40 °C schmilzt, und das in polaren Lösungsmitteln löslich ist.

Gemäß einer weiteren bevorzugten Ausführungsform umfasst der Stoffblister eine niedermolekulare, in Wasser lösliche Substanz g) mit einer endothermen Lösungs- bzw. Sorptionsenthalpie in Wasser.

Gemäß einer weiteren bevorzugten Ausführungsform besitzt die niedermolekulare, in Wasser lösliche Substanz g) eine endotherme Sorptions- bzw. Lösungsenthalpie in Wasser von 4.0 kJ/mol - 210 kJ/mol (entsprechend ca. 1 kcal/mol - 50 kcal/mol), vorzugsweise 40 kJ/mol - 100 kJ/mol.

Gemäß einer weiteren bevorzugten Ausführungsform enthält die niedermolekulare, in Wasser lösliche Substanz g) Harnstoff, sekundäres Natriumphosphat oder Kaliumnitrat.

Gemäß einer weiteren bevorzugten Ausführungsform umfaßt die Matrix c) eine poröse faser- oder schaumstoffartige Matrix.

Gemäß einer weiteren bevorzugten Ausführungsform umfaßt die Matrix c) ein Faservlies aus natürlichen und/oder synthetischen Fasern, insbesondere ein Polypropylenvlies.

Gemäß einer weiteren bevorzugten Ausführungsform ist der Duftstoff d) verkapselt bzw. eingekapselt (= inkapsuliert), wobei das Inkapsulierungsmittel vorzugsweise Cyclodextrin, Glukan, Chitosan und/oder Dendrimere umfaßt.

Gemäß einer weiteren bevorzugten Ausführungsform ist der Duftstoff d) vorzugsweise in Kombination mit Tensiden als Vesikel bzw. Liposom und/oder Mikrokapsel formuliert.

Gemäß einer weiteren bevorzugten Ausführungsform enthält das Polymer e) mindestens ein vernetzbares Polypeptid, z.B. Gelatine, Polysaccharid, z.B. Methocell, Dow Chemical Company, Hydroxyethyl Stärke, Suchema AG, Polyvinylalkohol, z.B. Mowiole Hoechst AG, oder Blockpolymerisat auf der Basis von Polyvinylalkohol und Polyvinylacetat / Polyvinylpyrrolidon, z.B. PVP / VA S-630 von ISP Global Technologies.

Gemäß einer weiteren bevorzugten Ausführungsform bildet das Polymer f) mit dem Polymeren e) und / oder dem Duftstoff d) eine homogene Phase, eine wässrige Dispersion oder eine Emulsion.

Gemäß einer weiteren bevorzugten Ausführungsform enthält das Polymer f) ein Ethylen /Propylenoxid - Blockpolymerisat, ein Fettsäurewachs, und/oder ein Polyethylenwachs.

Gemäß einer weiteren bevorzugten Ausführungsform enthält das Polymere f) Pluronic PE 9400, und / oder Pluronic PE 10500 (BASF).

Gemäß einer weiteren bevorzugten Ausführungsform enthält der Stoffblister mindestens ein Lösungsmittel, und/oder mindestens ein Verdickungs- und/oder Geliermittel, und/oder mindestens ein Vernetzungsmittel, und/oder mindestens ein Konservierungsmittel, und/oder mindestens einen grenzflächenaktiven Stoff.

Eine Ausführungsform der vorliegenden Erfindung betrifft ein Stoffdispensersystem, das mindestens einen Druckblister und/oder mindestens einen Stoffblister umfaßt.

Eine weitere Ausführungsform der Erfindung betrifft die Verwendung des Stoffdispensersystems zur Abgabe von Duftstoffen und/oder Arzneimitteln.

### Detaillierte Beschreibung

In der gesamten Anmeldung sind % Gewichtsprozente und Teile Gewichtsteile, sofern nicht anders angegeben.

### Verschlussmechanismus mittels Druckblister

Ein den Stoffdispenser charakterisierendes Novum ist der sich eigendynamisch regulierende physikalisch/chemische Verschlussmechanismus durch einen Druckblister (= Druckbehälter). Der Druckblister enthält ein Lösungsmittel bzw. Lösungsmittelgel b), dessen Dampfdruck in dem Druckblister einen temperaturabhängigen Druck erzeugt. Der bei Temperaturerhöhung - zunehmende Druck wird zur Auslösung des Verschlussmechanismus benutzt.

Die chemische Zusammensetzung der in dem Druckblister einzusetzenden Gelmasse, d.h. das verdickte bzw. sorbierte Lösungsmittel b), orientiert sich an der zur Schliessung des Systems notwendigen Kraft und somit an der individuellen Konstruktion des Duftstoffdispensers.

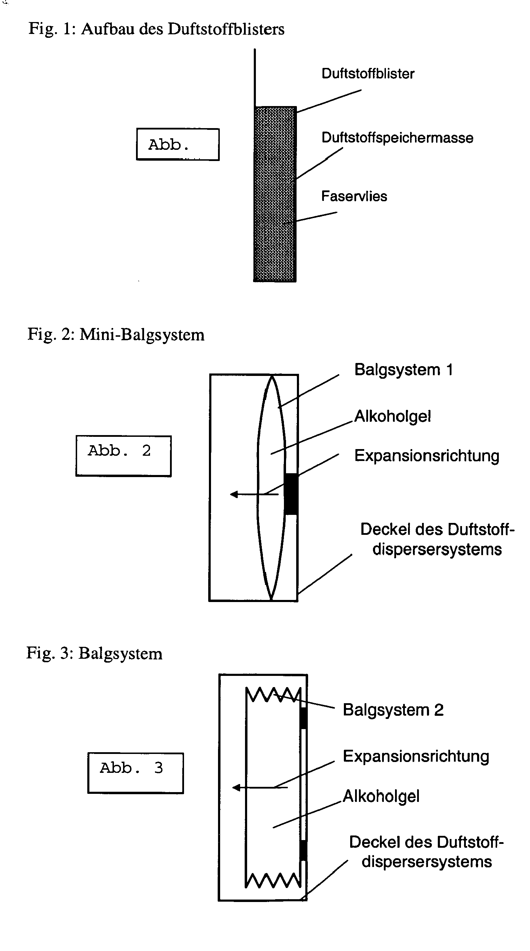

Die Konstruktion des **Behältnisses a)** hängt ebenfalls vornehmlich vom Verwendungszweck ab, und kann so konzipiert sein, dass die druckbedingte Ausdehnung des Behältnisses a) nur eindimensional ist. Es sind aber auch Ausdehnungen in zwei oder drei Dimensionen möglich. Als Beispiele für das Behältnis a) kommen z.B. ein Minibalgsystem (Fig. 3) oder ein rotationssymetrischer, sich nur eindimensional ausdehnender Ballon (Fig. 2) in Betracht. Die Ausdehnungsdistanzen des Druckblisters in der gewünschten, zum Verschliessen des Duftstoffblisters notwendigen Richtung betragen z.B. 1 - 20 mm, vorzugsweise 5 - 10 mm. Der das Druckgel beinhaltende Blister kann u.a. aus elastischen, gegenüber den verwendeten Lösungsmitteln beständigen Kunststoffen aufgebaut sein, die gegebenenfalls z.B. durch eine Aluminiumschicht für Lösungsmitteldampf undurchlässig gemacht sind.

Das Kernstück des Druckblisters ist mindestens ein in mindestens einer Dimension ausdehnungsfähiges Behältnis, in dem sich ein niedrig siedendes, vorzugsweise bei 40 - 100 °C siedendes **Lösungsmittel bzw. Lösungsmittelgemisch b)** befindet. Steigt infolge einer höheren Umgebungstemperatur der Dampfdruck des Lösungsmittels im Druckgefäss an, so dehnt sich dieses aus und verschliesst die Öffnung für die Abgabe der Duftstoffe mechanisch; die Abgabe der Duftstoffe wird somit mit dem Überschreiten einer bestimmten Temperatur unterbunden, die von der Zusammensetzung des Gemisches im Druckgefäss abhängt.

Zur Regulierung des von der Umgebungstemperatur abhängigen Dampfdrucks werden vorzugsweise Gemische von einzelnen Lösungsmitteln b) eingesetzt, die sich durch verschiedene Siedepunkte und/oder Verdampfungsenthalpien unterscheiden. Weitere den Dampfdruck bestimmende mögliche Zusätze sind der Polarität der Lösungsmittel angepasste Polymere, z.B. Poly(Methyl vinyl ether / Maleinsäure) "Gantrez SP-215", oder Lösungsmittel sorbierende Partikel, z.B. methylierte Silizium-Nanopartikel "Aerosil R 972" der Firma Degussa, die in Kombination mit dem Lösungsmittel Gele ausbilden.

Als geeignete Lösungsmittel für den Druckblister kommen bspw. Wasser, Methanol, Ethanol, Isopropanol, und Aceton in Betracht; insbesondere werden Alkohol-/Wassermischungen eingesetzt.

Als Beispiele für Verdickungsmittel für den Druckblister kommen Cellulosederivate und Polyvinylpyrrolidon in Betracht; insbesondere werden Hydroxypropyl-Methylcellulose (entspricht Methocell von Dow-Chemical Company), Ethylhydroxyethyl-Cellulose (entspricht Bermocoll E 230 FQ von AKZO Nobel) oder Polyvinylpyrrolidon, z.B. PVP K-120 der Firma ISP, als Verdickungsmittel eingesetzt. Die hierfür eingesetzten Polymerkonzentrationen betragen z.B. 0.5 - 10 %, vorzugsweise 1 - 5 %, bezogen auf die gesamte Gelmasse.

Als typische Lösungsmittel sorbierende anorganische Substanzen sind z.B. unterschiedlich modifizierte Siliziumdioxid-Nanopartikel der Firma Degussa AG, z.B. methylierte Silizium-Nanopartikel "Aerosil R 972" der Firma Degussa, zu erwähnen.

### Stoffblister

Der Stoffspeicher, auch als Stoffblister, oder als Stoffbehälter bezeichnet, umfasst mehrere Komponenten, die ihrerseits Träger der zur Zielerfüllung notwendigen Funktionen sind.

Als eine erste Komponente ist mindestens eine poröse **Matrix c)** als mechanisches "Gerüst" des Stoffspeichers zu bezeichnen. Diese Matrix ist z.B. ein poröses faser- oder schaumstoffartiges Flächengebilde bzw. Matrix. Vorzugsweise werden Faservliese mit einer Dicke von 1 bis 10 mm aus nativen und/oder Synthesefasern verwendet, die mit der Duftstoffträgermasse z.B. besprüht, getränkt oder beschichtet werden (siehe Fig. 1). Besonders bevorzugt ist eine poröse Matrix aus Polypropylenvlies, z.B. von der Firma Freudenberg AG, oder von der Firma Sandler AG. Es ist auch möglich, mehrere, auch beschichtete Vlieslagen als gestapeltes Paket zu einem Duftstoffblister zu konfektionieren.

Die zweite Komponente für den Stoffblister umfasst einen Duftstoff oder eine **Duftstoffmischung d)**. Bezüglich der Wahl der Duftstoffe ist das beschriebene System völlig offen, so dass allein der Anwendungsbereich des Duftstoffdispensers für die Auswahl der Duftsubstanz bestimmend ist. Insbesondere werden Duftstoffe aus der Terpenklasse verwendet, wie zum Beispiel Citronellol, Citronellal, Hydroxycitronellol, Hydroxycitronellal, Nerolidol, Linalool.

Der Stoffblister kann auch ein oder mehrere Arzneimittel enthalten, das/die entweder allein oder in Verbindung mit einem oder mehreren Duftstoffen als "Wirkstoff" eingesetzt werden kann/können. Bevorzugt werden Arzneimittel mit beruhigender Wirkung eingesetzt, wobei der Bedarf solcher Stoffdispensersysteme speziell im Krankenhausbereich liegt. Als Beispiel für ein Arzneimttel kann Happybelle PE dienen, ein Phyto-endorphin Extrakt der Fa. Mibelle AG, welcher analgetisch und euphorisierend wirkt, und somit zum allgemeinen Wohlbefinden beiträgt. Weiter für diesen Bereich einsetzbare Substanzen sind: Mandarinen-, Orangen- und Melissenöl sowie Hopfenöl und Passionsblumenextrakt, wobei letztere zwei sedierende Wirkung besitzen.

Durch die Inkapsulierung der Duftstoffe bzw. Arzneimittel kann deren Abgabegeschwindigkeit verzögert bzw. vergleichmässigt werden (Stoffabgabe gemäss einer Reaktion nullter Ordnung). Zusätzlich können dadurch auch weitere, unterschiedliche Funktionalitäten, z.B. allgemeines Wohlbefinden durch Happybelle PE der Fa. Mibelle AG, integriert werden. Zur Inkapsulierung der Duftstoffe bzw. Arzneimittel können Cyclodextrine, Glukan, Chitosan und/oder Dendrimere verwendet werden. Ebenso können die Duftstoffe bzw. Arzneimittel in Kombination mit Tensiden als Vesikeln, Liposomen, Nano- oder Mikropartikel formuliert werden.

Eine weitere Möglichkeit, eine Stoffabgabe von näherungsweise nullter Ordnung zu erzielen, ist die Emulgierung der Duftstoffe bzw. Arzneimittel in mindestens ein geschmolzenes Polymer f) (siehe unten) mittels grenzflächenaktiver Stoffe (= Tenside). Durch die langsame Abkühlung der so entstandenen Emulsion verfestigt sich diese unter Ausbildung von nanoskopischen "Containern", in denen der Duftstoff oder die Substanzen mit einer anderen Funktionalität eingeschlossen sind, z.B. Beruhigungsmittel. Als optional einsetzbare grenzflächenaktive Stoffe (= Tenside) zur Emulgierung werden z.B. ethoxylierte Fettalkohole, Glycerinester oder sulfonierte Lauryl- oder Stearylderivate eingesetzt. Der HLB-Wert (= Hydrophilic-Lipophilic Balance) dieser Substanzen bewegt sich im Bereich von 8 ― 18, bevorzugt 9 ― 16, insbesondere bevorzugt 10 ― 14.

Eine weitere Komponente des Stoffblisters ist ein **Polymer e)**, dessen Schmelz- bzw. Zersetzungstemperatur über 100°C liegt. Die Funktion dieses Bestandteils des Stoffblisters betrifft die chemische Stabilisierung der Duftstoff- bzw. Arzneimittel-Trägermasse, um bei erhöhten Temperaturen ein Fliessen der Trägermasse zu verhindern.

Als hierzu geeignete Substanzen für das **Polymer e)** werden vorzugsweise Substanzen eingesetzt, die nicht unter 100 °C schmelzen bzw., die sich erst bei einer Temperatur von oberhalb von 100°C zersetzen. Hierfür geeignet sind beispielsweise chemisch vernetzbare Polypeptide, zum Beispiel Gelatine; Polysaccharide, z.B. Methocell, Dow Chemical Company, Hydroxyethyl Stärke, Suchema AG; Polyvinylalkohole, z.B. Mowiole Hoechst AG; oder Blockpolymerisate auf der Basis von Polyvinylalkohol und Polyvinylacetat / Polyvinylpyrrolidon, z.B. PVP / VA S-630 von ISP Global Technologies. Die Einsatzmenge des Polymers e) liegt je nach verwendetem Polymer f) bei 20 - 150 g/l des wässrigen Gels, bezogen auf die Gelmasse des Stoffblisters.

Eine weitere Komponente für den Stoffblister betrifft das **Polymer f),** welches bei einer niedrigen Temperatur, insbesondere bei 23 bis 40°C, insbesondere bevorzugt bei 25 ― 30°C schmilzt. Insbesondere besitzt dieses Polymer f) eine hohe Schmelzenthalpie. Als Schmelzenthalpie wird die bei der Phasenumwandlung vom festen in den flüssigen Aggregatzustand umgesetzte so genannte "latente" Wärme bezeichnet. Die Schmelzenthalpie der hierzu benutzten Polymere liegt z.B. im Bereich von 50 - 500 J/g Polymer, vorzugsweise im Bereich von 150 - 250 J/g Polymer. Bei monomolekularen Stoffen werden die thermodynamischen Daten wie Schmelzethalpie oder Lösungs- bzw. Sorptionsenthalpie in cal/mol bzw.J/mol angegeben, bei polymeren Stoffen dagegen in cal/g Polymer bzw. J/g Polymer.

Das Polymer f) dieser Erfindung dient der Duftstoffaufnahme und/oder Arzneimittelaufnahme, Speicherung und "Wärmeregelung" - im Sinne eines "Temperaturpuffers" - des Stoffblisters. Es umfasst bei niederer Temperatur schmelzende, tendenziell lipophile Polymere mit einer möglichst hohen Schmelzenthalpie. Dies hat zur Folge, dass trotz erhöhter Umgebungstemperatur die Temperatur des Stoffblisters während einer längeren Zeit nicht über die Schmelztemperatur der zur Temperaturkontrolle eingesetzten Polymere ansteigt, da aufgrund der hohen Schmelzenthalpie eine grosse Wärmemenge für den Phasenübergang von fest nach flüssig erforderlich ist. Dadurch kann der Temperaturanstieg im Stoffblister aufgrund einer hohen Umgebungstemperatur gebremst werden.

Das Polymer f) ist ein bei niederen Temperaturen, vorzugsweise bei 23 - 40 °C, bevorzugt bei 25 ― 30°C schmelzbares Polymer, welches aufgrund seiner chemischen Konstitution sowohl in wässrigen als auch in polaren Lösungsmitteln löslich ist und mit dem Polymer e) und / oder dem Duftstoff bzw. Arzneimittel d) und ggf. der niedermolekularen, wasserlöslichen Substanz g) entweder eine homogene Phase oder eine wässrige Dispersion bzw. Emulsion ausbildet.

Bevorzugt als **Polymer f)** eingesetzte Substanzen sind bei 23 - 40 °C, vorzugsweise bei 25 ― 30°C schmelzbare Polymere wie z.B. Ethylen-/Propylenoxidblockpolymerisate oder Fettsäure- und Polyethylenwachse. Bevorzugt werden Pluronic PE 9400 und Pluronic PE 10500 (beides sind Markennamen der BASF und bezeichnen Ethylenoxid-Propylenoxid-Polymere) als Polymer f) verwendet.

Die Temperaturpufferkapazität wird einerseits durch die chemische Natur der Polymersubstanzen und deren Schmelzenthalpie, andererseits durch den eingesetzten Anteil des Polymers bestimmt. Die zu diesem Zweck eingesetzten Polymerkonzentrationen liegen z.B. bei 30 - 70 Gew.-%, bezogen auf die gesamte Duftstoffspeichermasse.

Aufgrund des breiten Löslichkeitsspektrums der in dieser Komponente zusammengeführten Ingredienzien sind die vorwiegend lipophilen Duftstoffe bzw. Arzneimittel direkt oder in Kombination mit grenzflächenaktiven Stoffen mischbar. Als grenzflächenaktive Stoffe werden z.B. ethoxylierte Fettalkohole, Glycerinester oder sulfonierte Lauryl- oder Stearylderivate eingesetzt. Der HLB-Wert (Hydrophilic-Lipophilic Balance) dieser Substanzen bewegt sich im Bereich von 8 - 18, bevorzugt von 9 - 16, insbesondere bevorzugt von 10 - 14.

Ein weiterer Bestandteil der Mischung für den Stoffblister, der vorzugsweise enthalten sein kann, umfasst eine **niedermolekulare, wasserlösliche Substanz g) mit einer endothermen Lösungs- bzw. Sorptionsenthalpie** in Wasser. Als Lösungsenthalpie wird jene Wärmemenge [kcal/mol] bezeichnet, die z.B. beim Lösen einer Substanz in Wasser frei wird (exotherm) oder aufzuwenden (endotherm) ist. Als Sorptionsenthalpie eines Stoffes bezüglich Wasser bezeichnet man jene Wärmemenge [kcal/mol bzw. kJ/mol], die entweder freigesetzt wird (= exotherm) oder aufgewendet werden muß (= endotherm), wenn Wasser bzw. Wasserdampf von diesem Stoff, der mit dem Wasserdampf in Berührung steht, aufgenommen wird (Römpp: Chemie Lexikon, Band 5, Thieme, Stuttgart 1992).

Die niedermolekulare, wasserlösliche Substanz g) mit einer endothermen Lösungs- bzw. Sorptionsenthalpie in Wasser dient dazu, das Wasser der Umgebungsluft zu sorbieren und durch die endotherme Sorptionsenthalpie die Temperatur des Stoffblisters mit der Trägermasse zu senken. Solche Substanzen mit endothermer Lösungs- bzw. Sorptionsenthalpie wirken in der vorliegenden Mischung für den Stoffblister als "Temperaturpuffer" in dem Sinne, dass sie einen Anstieg der Temperatur der Masse in dem Stoffblister verhindern bzw. die Temperatur der Masse in dem Stoffblister herabsetzen, wenn die (absolute) Luftfeuchtigkeit der Umgebung, und damit in der Regel auch die Temperatur der Umgebung zunimmt.

Die hohe Schmelzenthalpie des Polymers f) sowie die endotherme Sorptions- bzw. Lösungsenthalpie in Wasser der niedermolekularen, wasserlöslichen Substanz g) der Stoffzusammensetzung für den Stoffblister sind beide für den "Temperaturpuffer" verantwortlich, aufgrund dessen die von der Umgebungstemperatur relativ unabhängige, gleichmässige Abgabe der Duftstoffe aus der Mischung erfolgt. Sie wirken insofern in die gleiche Richtung, d.h. synergistisch.

Beispiele für eine niedermolekulare, wasserlösliche Substanz g) mit einer endothermen Lösungs- bzw. Sorptionsenthalpie in Wasser sind Harnstoff mit einer Lösungsenthalpie von 15,5 kJ/mol (bzw. 3,7 kcal/mol) sowie dessen Derivate, aber auch anorganische Salze wie z.B. Dinatriumhydrogenphosphat (Na₂HPO₄ · 12 H₂O) mit einer Lösungsenthalpie von 93,9 kJ/mol (bzw. 22,4 kcal/mol), oder Kaliumnitrat (KNO₃). Die Einsatzmengen typischer, Wasser-sorbierender Mittel mit endothermer Lösungswärme liegen bei 0,1 - 10 Gew.-%, bevorzugt bei 0,2 ― 5,0 Gew.-%, besonders bevorzugt bei 0,2 ― 2,0 Gew.-%, insbesondere bevorzugt bei 0,2 ― 1,0 Gew.-%, bezogen auf die gesamte Duftstoffspeichermasse.

Die temperaturunabhängige, gleichmässige Duftstoff- bzw. Arzneimittel-Abgabe, basierend auf dem beschriebenen Funktionsprinzip erlaubt es, auf der Stoffblisterhülle eine für den Benutzer sichtbare Markierung anzubringen, die ein zur noch im Stoffblister vorhandenen Duftstoff- bzw. Arzneimittelmenge proportionales Signal abgibt, um so dem Benutzer den Zeitpunkt des Blisteraustausches zu markieren. Möglichkeiten hierzu bestehen z.B. in der Applikation beschränkt lichtechter oder sauerstoffunbeständiger Farbstoffe, deren Ausbleichungszeit mit der Duftstoffspeicherkapazität korreliert.

Darüber hinaus kann der Stoffblister mindestens ein Lösungsmittel enthalten. Beispiele hierfür sind Wasser, Methanol, Ethanol, Isopropanol und Aceton, insbesondere Wasser und Methanol.

Darüber hinaus kann der Stoffblister mindestens ein Verdickungs- und / oder Gelierungsmittel enthalten. Beispiele hierfür sind Gelatine, Cellulose- und Guar-Derivate, Alginate und Polyvinylpyrrolidon.

Darüber hinaus kann der Stoffblister mindestens ein Vernetzungsmittel enthalten. Beispiele hierfür sind Glutaraldehyd, Formaldehyd, Trioxan, methoxylierte Ethylenharnstoff- und Melaminderivate.

Darüber hinaus kann der Stoffblister mindestens ein Konservierungsmittel enthalten. Beispiele hierfür sind Harnstoff ― Peroxid ― Additionsprodukt, Imidazolidinylharnstoff, Diazolidinylharnstoff, Methylparaben, Propylparaben.

Darüber hinaus kann der Stoffblister mindestens einen grenzflächenaktiven Stoff enthalten. Beispiele hierfür sind ethoxylierte Fettalkohole, Glycerinester oder sulfonierte Lauryl- oder Stearylderivate. Der HLB-Wert (Hydrophilic-Lipophilic Balance) dieser Substanzen bewegt sich im Bereich von 8 ― 18, bevorzugt von 9 ― 16, insbesondere bevorzugt von 10 ― 14.

Die Herstellung des Stoffblisters geschieht durch das Zusammenfügen bzw. Einmischen der Komponenten d), e), f) und ggf. g) zu einer Masse, die auf die poröse Matrix c) appliziert wird. (siehe Beispiele). Anschließend wird bei Anwesenheit von Lösungsmitteln nach dem Auftragen auf die Matrix c) ein Trocknungsprozess nachgeschaltet, so dass zumindest ein Teil des Lösungsmittels verdunstet.

Die gegenseitige mechanische Unabhängigkeit des Stoffblisters und des Druckblisters machen es möglich, dass sowohl das gesamte Stoffdispensersystem als Wegwerfartikel als auch als nachfüllbares System mit den beiden Systemkomponenten (Stoff- bzw. Druckblister) gefertigt werden kann. Diese Möglichkeit birgt den Vorteil, Designer-Systeme herzustellen, deren Stoffblister durch andere Stoffblister z.B. mit zusätzlichen Funktionalitäten austauschbar sind.

Als Gehäuse für Druckblister und Stoffblister ist jegliches Behältnis geeignet, das zumindest einen Druckblister und zumindest einen Stoffblister aufnehmen kann. Beispiele derartiger verschliessbarer Behältnisse sind Spritzgussboxen oder auch extrudierte oder auf andere Weise geformte Kunststoffboxen.

### Beispiel 1: Duftstoffdispenser für Autos mit Druckblister

Zur Herstellung eines Duftstoffdispensers für Autos wird eine verschliessbare Spritzgussbox benötigt, in der sich ein auswechselbarer Duftstoffblister und ein in das Spritzgussteil integrierter Druckblister zur Temperatur bedingten Schliessung des Duftstoffdispensors befinden.

Die Spritzgussbox ist so konzeptiert, dass sich durch eine Drehung des Spritzgussdeckels zwei einander gegenüberliegende Schlitze des Duftstoffdispensors (unten und oben) öffnen. Durch die geöffneten Schlitze kann die Umgebungsluft zwischen dem Duftstoff- und Druckblister hindurchströmen, den Duftstoff aufnehmen, der dann durch die konvektive, im Auto vorherrschende Luftströmung, verteilt wird.

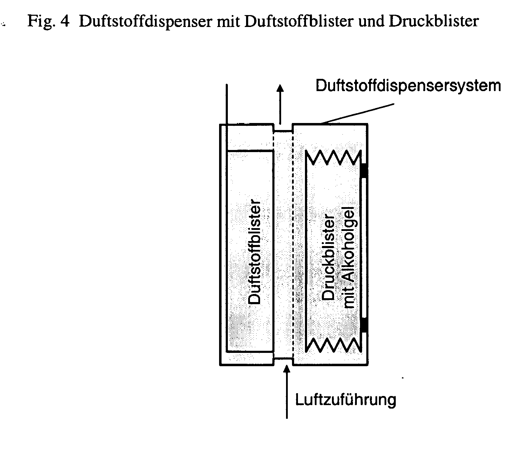

Bei zunehmender thermischer Belastung (z.B. bei starker Sonneneinstrahlung) erhöht sich der Dampfdruck im Druckblister, der sich in Folge in Richtung des Duftstoffblisters bis zu dessen Schliessung ausdehnt. Beim Absinken der Temperatur zieht sich auch der Druckblister wieder zurück und öffnet so erneut den Luftkanal (siehe Fig. 4).

### Herstellung des Duftstoffblisters:

Die den Duftstoff beinhaltende Polymermasse enthält folgende Ingredienzien:

| **Bestandteil** | **Funktion** | **Anteil (Gew.-%)** |
|---|---|---|
| Hydroxycitronellal | Duftstoff | 24% |
| Pluronic PE 9400 | Blockpolymerisat (BASF) mit hoher Schmelzenthalpie | 21 % |
| Pluronic PE 10500 | Blockpolymerisat (BASF) mit hoher Schmelzenthalpie | 10 % |
| Methanol | Lösungsmittel | 5 % |
| Kaliumnitrat | Niedermolekulare, wasserlösliche Substanz mit endothermer Lösungs- bzw. Sorptionsenthalpie | 1 % |
| Gelatine | Stabilisierende Polypeptide, das sich bei mehr als 100°C zersetzt; bzw. Verdicker u. Gelierungsmittel | 4% |
| Glutaraldehyd | Vernetzer | 0.5% |
| Methylparaben | Konservierungsmittel | 0.5 % |
| Wasser | Lösungsmittel | 34 % |

Ein Polypropylenfaservlies mit einem Quadratmetergewicht von 180 g wird beidseitig mit dieser den Duftstoff enthaltenden Polymermasse beschichtet. Das beschichtete Vlies wird den Ausmassen des Blisters entsprechend in 40 x 40 mm Stücke gestanzt, in einen Blistersack eingebettet und verschweisst. Die Verschlussfolie wird unmittelbar vor dem Blistergebrauch abgezogen und der Blister in das Spritzgussteil eingeschoben.

### Herstellung des Druckblisters

Das bei thermischer Belastung den Druck erzeugende Alkoholgel besteht aus folgenden Ingredienzien:

| **Bestandteil** | **Anteil** | **Einheit** |
|---|---|---|
| Methanol | 890 | g/kg |
| Wasser | 100 | g/kg |
| Methocell | 10 | g/kg |

Ein zylindrischer aus Kunststoff bestehender Faltenbalg wird mit 3 g dieses Alkoholgels beschickt und dicht verschlossen (verschweisst). Die Fixierung des Druckblisters erfolgt auf der zum Duftstoffblister abgekehrten Seite, durch punktuelle Verschweissung mit dem Rand des Duftstoffdispensers (siehe Fig. 4).

Der beschriebene Duftstoffdispenser zeichnet sich durch eine sehr gute Wärmestabilisierung bei 30 - 35 °C aus. Der Druckblister ist in der Lage, bei ca. 35 - 40 °C innerhalb von 6 Minuten den Luftkanal zwischen Duftstoff- und Druckblister (ca. 5 mm) zu schliessen. Durch diese Massnahme ist eine weitere Duftstoffabgabe unter erhöhten Temperaturen weitgehend verhindert.

### Beispiel 2: Duftstoffdispenser für den Krankenhausgebrauch ohne Druckblister

Der Einsatz des hier beschriebenen Duftstoffdispensers ist dann angebracht, wenn parallel zur "Wellness" verbreitenden Funktion auch eine den Patienten sedierende Wirkung erzielt werden soll. Dies ist z.B. dann der Fall, wenn ein Patient ein agitives, aber auch ein gegenüber dem Betreuungspersonal oder den Mitpatienten agressives Verhalten zeigt. Die hier eingesetzten Duftstoffdispenser unterscheiden sich von dem im Beispiel 1 beschriebenen durch das Fehlen des Druckblisters und die Zusammensetzung der Duftstoffspeichermasse und den damit verbundenen Funktionen des Duftstoffblisters.

### Herstellung der Duftstoffspeichermasse zur Beschichtung des Faservlieses.

30 g in Wasser verquollenes Glukan wird in eine Ethanollösung eingerührt, die das Beruhigungsmittel Happybelle PE der Fa. Mibelle AG in einer Konzentration von 50 g/l enthält. Nach zweistündiger Verweildauer des Glukans in der ethanolischen "Happybelle PE"-Lösung wird das Glukan zentrifugiert und in einem geschlossenen Gefäss aufbewahrt, bis zu dessen Einmischung in die den Duftstoff enthaltende Polymermasse. Die Polymermasse besteht aus 15 % (bezogen auf die gesamte Duftstoffspeichermasse) eines Fliederöls, 25 % Pluronic PE 10500 (BASF), 25 % Pluronic PE 9400 (BASF), und 5 % Methanol. Diese Mischung zeigt einen Schmelzbereich von 30 ― 40°C.

Die erwähnten 15 % des Duftstoffes werden mit der geschmolzenen Polymermasse vermischt und homogenisiert. Anschliessend wird das das Beruhigungsmittel enthaltende Glukan in die Polymerschmelze eingerührt. Nach erfolgter Dispergierung wird das Polymergemisch in eine wässrige Gelatinelösung eingemischt, bestehend aus 150 g/l Gelatine, 20 g/l Dinatriumhydrogenphosphat und 10 g/l eines Konservierungsmittels. Das Mischungsverhältnis zwischen der Duftstoff enthaltenden Polymerlösung und der Gelatinelösung beträgt 70 % zu 30 %.

### Eine typische Rezeptur für einen Krankenhausblister ist in der nachfolgenden Tabelle gezeigt:

| **Bestandteil** | **Funktion** | **Anteil (Gew.-%)** |
|---|---|---|
| Glukan+Happybelle | Mikrokapsel+"Wellnessstoff" | 21 % |
| Pluronic PE 9400 | Blockpolymerisat (BASF) mit hoher Schmelzenthalpie | 17.5 % |
| Pluronic PE 10500 | Blockpolymerisat (BASF) mit hoher Schmelzenthalpie | 17.5 % |
| Methanol | Lösungsmittel | 3.5 % |
| Fliederöl | Duftstoff | 10.5% |
| Dinatriumphosphat | Niedermolekulare, wasserlösliche Substanz mit endothermer Lösungs- bzw. Sorptionsenthalpie | 0.6 % |
| Gelatine | Stabilisierendes Polypeptide als Verdicker u. Gelierungsmittel | 4.5 % |
| Germaben II-E | Konservierungsmittel (Diazolidinylharnstoff+Methyl- und Propylparaben, Firma ISP) | 0.3 % |
| Wasser | Lösungsmittel | 24.6% |

Die so hergestellte Dispersionsmasse wird bei ca. 30 - 35 °C auf das Faservlies appliziert. Das beschichtete Faservlies wird anschliessend gestanzt und in der Blisterbox verschweisst. Die Benützung dieses Blisters ist analog der in Beispiel 1 beschriebenen, mit dem Unterschied, dass zwei Funktionen, eine "Wellness" verbreitende und eine sedierende, erfüllt werden.

Die so hergestellten Duftstoffdispensersysteme zeichnen sich durch eine gleichmässige, auch bei schwankenden Zimmertemperaturen nur wenig variierende Stoffabgabe aus. Der grosse Vorteil dieser Systeme ist deren Mehrfachfunktion, wobei die vom Patienten wahrgenommene nur die "Wellness"-Funktion ist.

## Patentansprüche

1. Druckblister für ein Stoffdispensersystem mit
a) einem in mindestens einer Dimension ausdehnungsfähigen Behältnis, das
b) mindestens ein niedrig siedendes Lösungsmittel enthält.

2. Druckblister nach Anspruch 1, wobei
das Lösungsmittel b) ein Verdickungsmittel und/oder ein Sorptionsmittel enthält.

3. Druckblister nach Anspruch 2, wobei
- das Verdickungsmittel ein Cellulosederivat und/oder Polyvinylpyrrolidon, und/oder wobei
- das Sorptionsmittel chemisch modifizierte Siliciumdioxid-Nanopartikel umfasst.

4. Druckblister nach Anspruch 2 oder 3, wobei das Verdickungsmittel eine Hydroxypropyl-Methylcellulose und/oder eine Ethylhydroxyethyl-Cellulose ist.

5. Stoffblister für ein Stoffdispensersystem mit
c) mindestens einer porösen Matrix, und
d) mindestens einem Duftstoff und/oder Arzneimittel, und
e) mindestens einem Polymer, das bei einer Temperatur von mehr als 100 °C schmilzt bzw. sich zersetzt, und / oder
f) mindestens einem Polymer, das bei einer Temperatur von 23 - 40 °C schmilzt, und das in polaren Lösungsmitteln löslich ist.

6. Stoffblister nach Anspruch 5 mit
g) einer niedermolekularen wasserlöslichen Substanz mit endothermer Lösungs- bzw. Sorptionsenthalpie.

7. Stoffblister nach Anspruch 6, wobei die niedermolekulare wasserlösliche Substanz g) Harnstoff, sekundäres Natriumphosphat oder Kaliumnitrat enthält.

8. Stoffblister nach einem der Ansprüche 5, 6 oder 7, wobei die poröse Matrix c) eine faser- oder schaumstoffartige Matrix ist.

9. Stoffblister nach einem der Ansprüche 5, 6, 7 oder 8, wobei die Matrix c) ein Faservlies aus natürlichen und/oder synthetischen Fasern, insbesondere ein Polypropylenvlies ist.

10. Stoffblister nach einem der Ansprüche 5 bis 9, wobei der Duftstoff d) inkapsuliert ist, und das Inkapsulierungsmittel vorzugsweise Cyclodextrin, Glukan, Chitosan und/oder Dendrimere umfaßt.

11. Stoffblister nach einem der Ansprüche 5 bis 10, wobei der Duftstoff d) als Vesikel, Liposom und/oder Mikrokapsel formuliert ist.

12. Stoffblister nach einem der Ansprüche 5 bis 11, wobei das Polymere e) mindestens ein vernetzbares Polypeptid, Polysaccharid, Polyvinylalkohol, oder Blockpolymerisat auf der Basis von Polyvinylalkohol und Polyvinylacetat / Polyvinylpyrrolidon enthält.

13. Stoffblister nach einem der Ansprüche 5 bis 12, wobei das Polymere f) mit dem Polymeren e) und/oder dem Duftstoff d) eine homogene Phase, eine wässrige Dispersion oder eine Emulsion bildet.

14. Stoffblister nach einem der Ansprüche 5 bis 13, wobei das Polymere f) ein Ethylen /Propylenoxid - Blockpolymerisat, ein Fettsäurewachs, und/oder ein Polyethylenwachs enthält.

15. Stoffblister nach einem der Ansprüche 5 bis 14, wobei das Polymere f) Pluronic PE 9400, und / oder Pluronic PE 10500 (BASF) enthält.

16. Stoffblister nach einem der Ansprüche 5 bis 15, wobei der Stoffblister
- mindestens ein Lösungsmittel, und/oder
- mindestens ein Verdickungs- und/oder Geliermittel, und/oder
- mindestens ein Vernetzungsmittel, und/oder
- mindestens ein Konservierungsmittel, und/oder
- mindestens einen grenzflächenaktiven Stoff enthält.

17. Stoffdispensersystem, das
- mindestens einen Druckblister nach einem der Ansprüche 1 bis 4, und / oder
- mindestens einen Stoffblister nach einem der Ansprüche 5 bis 16 umfaßt.

18. Verwendung des Stoffdispensersystems nach Anspruch 17 zur Abgabe von Duftstoffen und/oder Arzneimitteln.
